(19) Europäisches Patentamt

European Patent Office

Office européen des brevets

(11) **EP 2 287 590 A2**

(12) **EUROPEAN PATENT APPLICATION**

(43) Date of publication:
**23.02.2011 Bulletin 2011/08**

(51) Int Cl.:
***G01N 21/27*** *(2006.01)*

(21) Application number: **10158678.2**

(22) Date of filing: **31.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **20.08.2009 KR 20090077132**

(71) Applicant: **Samsung Electronics Co., Ltd.
Suwon-si, Gyeonggi-do (KR)**

(72) Inventors:
• **Cho, Hyug Rae**
  Seoul (KR)
• **Lee, Jong Rip**
  Kyungki-do (KR)
• **Lee, Sung Hwa**
  Gyeonggi-do (KR)

(74) Representative: **Grünecker, Kinkeldey,
Stockmair & Schwanhäusser
Anwaltssozietät
Leopoldstrasse 4
80802 München (DE)**

(54) **Method and apparatus for calibrating result from test device using a reagent**

(57)    A method and apparatus for calibration of a result from a test device using a reagent are provided. The method includes measuring an optical signal for a sample contained in the test device to obtain a measured optical signal value, the sample having a known concentration; and determining a relationship between the known concentration of the sample and a ratio of an estimated optical signal value to the measured optical signal value of the sample.

FIG. 4

**EP 2 287 590 A2**

**Description**

**BACKGROUND**

*1. Field*

Apparatuses and methods consistent with exemplary embodiments relate to calibrating a result from a test device using a reagent.

*2. Description of the Related Art*

In order to diagnose a disease in a patient, to monitor progress thereof, to detect effects of a treatment on the disease and to determine a prognosis of the disease, a biological sample (e.g., blood or urine) is taken from a patient and subjected to assay or analysis.

In order to conveniently conduct different tests upon a single sample at the same time, a variety of sample testers and/or analyzers exist in the related art.

For instance, for implementation of a biochemical or immuno-serum test for patient blood, a disc-type test device having a chamber filled with a test reagent has been developed. When a blood sample of a patient is introduced into the tester and the test device is placed in an analyzer, the reagent contained in the chamber of the test device reacts with the blood sample. A resultant product is then analyzed by any related art method of spectrophotometric analysis.

However, reagent used in the test device may vary in reactivity over time, thus causing measurement errors where a sample is analyzed a long time after manufacture of the test device.

**SUMMARY**

Exemplary embodiments provide an apparatus and a method for calibration of results obtained from a test device using a reagent.

Another exemplary embodiment provides a computer program product for conducting calibration of results from a test device using a reagent, the computer program product being embodied on a computer readable medium and executed by a computer.

Another exemplary embodiment provides an analytic device for analyzing a sample placed in a test device by spectrophotometric analysis and for calibrating results of the analysis.

According to an aspect of an exemplary embodiment, there is provided a method for calibration of results obtained from a test device using a reagent, the method including: introducing a sample with a known concentration into the test device and measuring an optical signal thereof; and defining a relationship between a concentration of the sample and a ratio of an estimated optical signal value from the above known sample concentration to the measured optical signal value.

The above calibration method may also include calculation of an optical signal value corresponding to a random concentration by an inverse function of a master calibration curve (MCC) and the defined relationship. The MCC will be described in detail below.

The method may further include correction of the MCC using the above optical signal value.

According to an aspect of another exemplary embodiment, there is provided a computer program product for conducting the above calibration method wherein test results from a test device using a reagent are calibrated, the computer program product embodied on a computer readable medium and executed by a computer to perform the method.

According to another aspect of an exemplary embodiment, there is provided an analytic device for analyzing a sample placed in a test device and for calibrating the analyzed results, which includes: an identification unit to recognize the test device; and an analysis unit to analyze the sample contained in the test device by spectrophotometric analysis. The analysis unit measures an optical signal for a sample with a known concentration, which was introduced into the test device, and defines a relationship between a concentration of the sample and a ratio of an estimated optical signal value from the known sample concentration to the measured optical signal value.

**BRIEF DESCRIPTION OF THE DRAWINGS**

These and/or other aspects will become apparent and more readily appreciated from the following description of the exemplary embodiments, taken in conjunction with the accompanying drawings of which:

FIG. 1 illustrates an example of a test device using a reagent, to which a calibration method according to an exemplary embodiment is applicable;

FIG. 2 is a graph illustrating an exemplary MCC for a specific reagent, according to an exemplary embodiment;

FIG. 3 is a graph illustrating a variation in the MCC over time, according to an exemplary embodiment;

FIG. 4 is a flow chart illustrating a method for calibration of results from the test device according to an exemplary embodiment;

FIGS. 5A and 5B are two graphical curves each illustrating a relationship between a concentration of a sample and a ratio of an estimated optical signal value to a measured optical signal value, according to an exemplary embodiment; and

FIG. 6 is a block diagram illustrating a configuration of an analytic device according to an exemplary embodiment.

## DETAILED DESCRIPTION

Hereinafter, reference will be made in detail to the exemplary embodiments without particular restriction to these embodiments. The following detailed description includes specific details in order to provide a thorough understanding of the exemplary embodiments. However, it will be apparent to those skilled in the art that the exemplary embodiments may be practiced without such specific details.

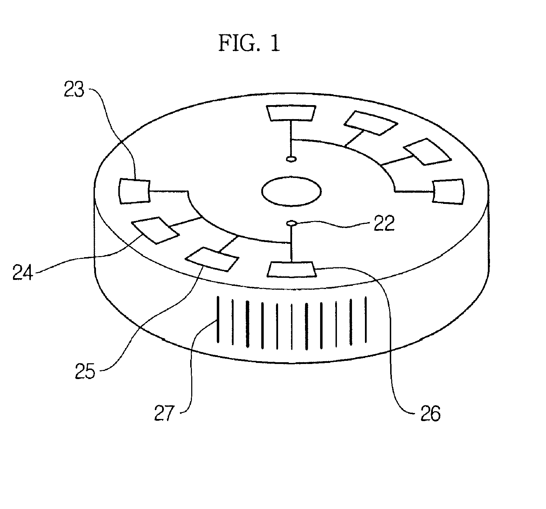

FIG. 1 illustrates an example of a test device using a reagent, to which a calibration method according to an exemplary embodiment is applicable. The test device is fabricated in the form of a centrifugally-rotatable disc, wherein a sample (e.g., a biological sample such as blood) is introduced through an input port 22 into a plurality of chambers 23, 24, 25 and 26. Each of the chambers 23, 24, 25 and 26 may contain a different reaction reagent with which the biological sample.

An analytic device such as a spectrophotometer uses a method of spectrophotometric analysis to irradiate a reaction product with light in order to analyze optical signals obtained therefrom, such as light absorbance or light transmittance, thus providing quantitative or qualitative analysis of a sample. On an outer side of the foregoing test device, a disc identifier (ID) is provided in the form of a barcode 27. The foregoing analytic device reads the barcode 27 to determine the disc ID of the test device and compares the disc ID to a pre-configured database to determine what kinds of tests are executed by the test device mounted on the analytic device and/or what kinds of reagents are contained in the chambers 23, 24, 25 and 26.

Commercially available testers or other testers under development have an MCC for a reagent contained therein. The MCC is a function expressing interrelation between a concentration of a sample and an optical signal measured for a reaction product where the sample has reacted with the reagent. When a test device is fabricated, an MCC is derived from combination of varied light absorbance and/or light transmittance of a reaction product at different concentrations of a sample. The derived MCC is provided with distribution of the test device. The MCC is defined in consideration and reflection of many variables in relation to the manufacture of test devices. Therefore, even among one model of test device, the MCC for individual devices may vary according to production lots.

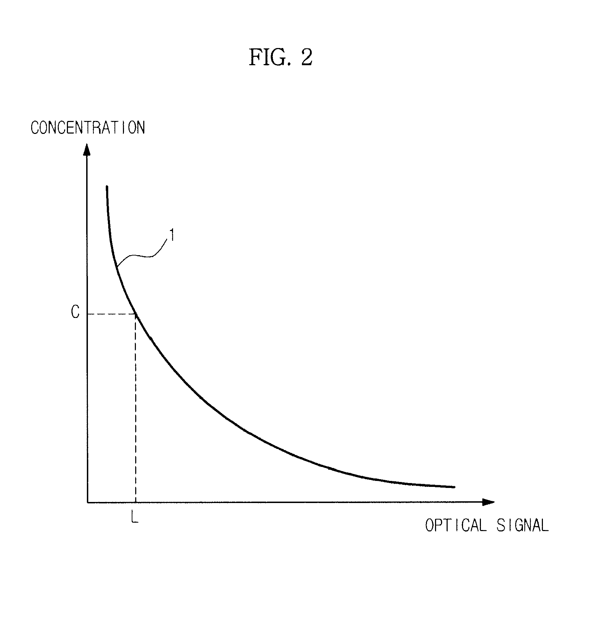

FIG. 2 is an exemplary MCC for a specific reagent. As illustrated in FIG. 2, an interrelation between a sample concentration and an optical signal (e.g., light absorbance) of a reaction product where the sample reacts with the reagent may be expressed by a curve 1. When the light absorbance of the reaction product is measured to be L during sample test, a concentration of a particular ingredient contained in the sample is presumed to be C.

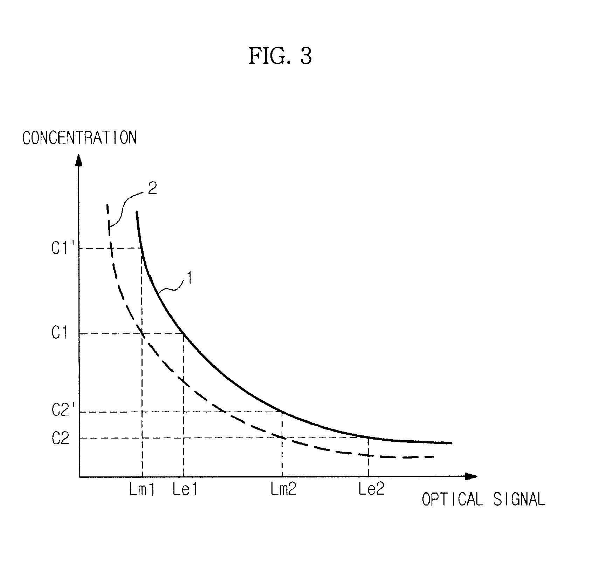

The reagent provided in the test device exhibits variation in reactivity over time and an extent of such variation may depend on the particular kind of reagent. For instance, as shown in FIG. 3, the MCC 1 exhibits reactivity which varies over time and, for example, moves toward another MCC 2 which represents an interrelation between a concentration and a light absorbance at a time of, e.g., 38 days after preparation of the reagent.

Accordingly, where samples at concentrations C1 and C2 are subjected to testing, these have light absorbance Le1 and Le2, respectively, according to the MCC 1 determined during production thereof. However, the light absorbance of the samples practically measured in the test device 38 days after production of the samples are Lm1 and Lm2, respectively, which are lower than expected values. More particularly, an examiner will understand that if the light absorbance practically measured in the test device is Lm1 after 38 days, a true concentration is C1. However, when the pre-determined MCC 1 is used, the concentration of the sample is estimated to be C1', thus causing a mathematical error between C1 and C1'.

Therefore, the pre-determined MCC 1 requires calibration at fixed periods or unfixed periods (that is, regularly or irregularly) over time.

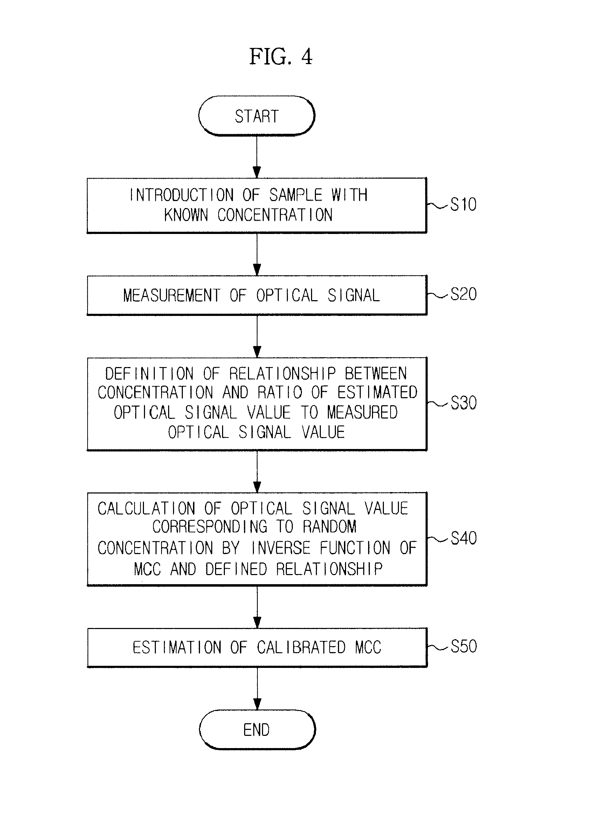

FIG. 4 is a flow chart illustrating a method of calibrating results from the test device according to an exemplary embodiment. In this exemplary embodiment, a sample with a known concentration is first introduced into the test device (S10), and then an optical signal thereof is measured (S20). Here, the sample substantially includes at least two samples with different concentrations. In the exemplary embodiment, only two samples are used to calibrate the test results, thereby satisfactorily reducing errors thereof. Consequently, the calibration method according to the exemplary embodiment may noticeably enhance accuracy of test results while minimizing inconvenience of an examiner.

After measurement of the optical signal, a relationship between a concentration of the sample and a ratio of an estimated optical signal value from the above known sample concentration to the measured optical signal value (i.e., estimated optical signal value/measured optical signal value) is defined (S30).

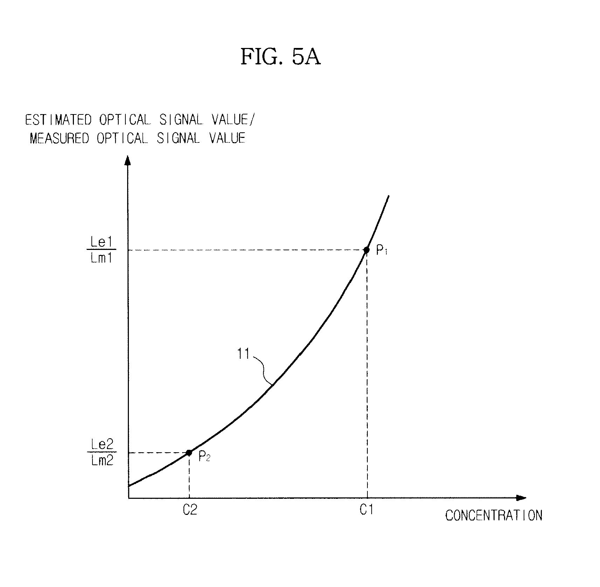

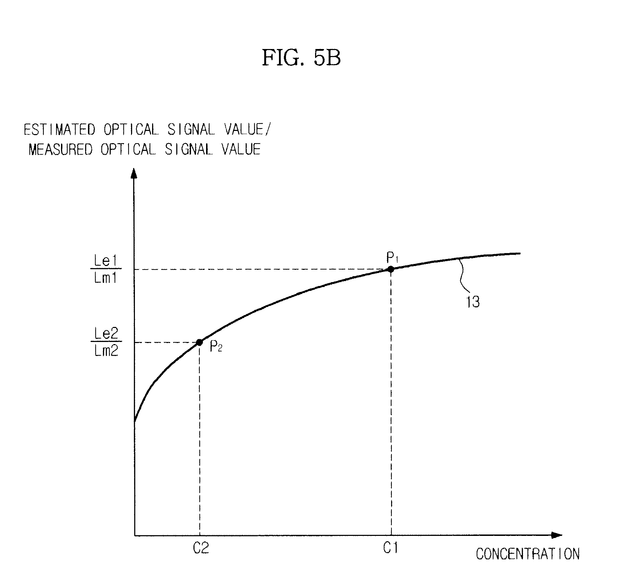

More particularly, as shown in FIGS. 3, 5A and 5B, an estimated optical signal value Le1 at concentration C1 (that is, an optical signal value calculated on MCC 1) and a measured optical signal value Lm1 at concentration C1 may be expressed by point P1 as shown in FIGS. 5A and 5B. Similarly, another estimated optical signal value Le2 at concentration C2 and another measured optical signal value Lm2 at concentration C2 may be expressed by point P2 as shown in FIGS. 5A and 5B. These values may be expressed by points P1 and P2, respectively, on a plane defined by an axis for a ratio of estimated optical signal value to measured optical signal value, and another axis for sample concentration, as illustrated in FIGS. 5A and 5B.

Connecting the points P1 and P2, two curves may be plotted. A first curve 11 is convex down as shown in FIG. 5A, while a second curve 13 is convex up as shown in FIG 5B. The convex-up curve 13 refers to an inversely proportional relationship between concentration and light absorbance or similar relations. On the other hand, the convex-down curve 11 represents a direct proportional relationship between concentration and light absorbance or similar relations. The convex-down curve 11 shown in FIG. 5A is applicable to quadratic or more polynomial equations or exponential function, however, exemplary embodiments are not particularly limited thereto.

Likewise, the convex-up curve 13 shown in FIG. 5B is applicable to a logarithmic function or root function, however, exemplary embodiments are not particularly limited thereto.

For a particular reagent, a test device manufacturer may preliminarily investigate a reagent in order to determine whether the reagent has the convex-up curve or the convex-down curve. The determined information may be input by reading a barcode (27 in FIG. 1) of a test disc containing the reagent and/or may be distributed with the new test device. If desired, an examiner may check characteristics of a particular reagent through alternative experimentation. Otherwise, a relationship between concentration and a ratio of estimated optical signal value to measured optical signal value may be automatically selected in a test device or an analytic device, based on characteristics of interrelation between concentration and optical signal.

For any one of the foregoing curve modes, the number of undetermined coefficients in the curve is substantially the same as the number of samples to be measured in the operations S10 and S20. For example, the convex-down curve illustrated in FIG. 5A, may be expressed as the following Equation 1:

$$[\text{Equation 1}] \qquad y = ax^2 + b$$

wherein y is a ratio of an estimated optical signal value to a measured optical signal value; x is a concentration; and a and b are undetermined coefficients.

A general quadratic curve expression has three undetermined coefficients, as shown in the following Equation 2:

$$[\text{Equation 2}]$$

$$y = ax^2 + bx + c$$

However, for the reagent used in the exemplary embodiment, the ratio of estimated optical signal value to measured optical signal value continuously rises as the concentration of the reagent increases. Thus, there is no case where b is less than 0 (b < 0). A curve connecting two points far apart from an axis of symmetry (i.e., x = -b/2a) is substantially linear to be almost a straight line and is not favorably used as a curve expression. Therefore, it is presumed that the absolute value of b is not large. In the exemplary embodiment, Equation 1 is applicable with b = 0.

Selection of an undetermined coefficient among coefficients of the curve expression depends on the number of samples.

For example, when three samples with different concentrations are measured during operations S10 and S20, a curve expression having three undetermined coefficients, e.g., Equation 2, may be used. This is also the same even when a cubic function or a logarithmic function other than a quadratic function is applied. Accordingly, upon application of a technique for selecting one among undetermined coefficients present on a curve expression then fixing the selected coefficient to a desired value, an assumption that an undetermined coefficient selected is correlated with (or combined with) another undetermined coefficient, etc. may enable completion of the curve expression such that the number of samples would be substantially the same as the number of undetermined coefficients.

Moreover, for example, where the convex-up curve is applied to the exemplary embodiment as illustrated in FIG. 5B, a curve expression based on logarithmic function having two undetermined coefficients is represented by the following Equation 3:

$$[\text{Equation 3}]$$

$$y = a \log (x + 1) + b$$

wherein y is a ratio of an estimated optical signal value to measured an optical signal value; x is a concentration; and a and b are undetermined coefficients.

In this exemplary embodiment, the above curve-type relationship will be referred to as a "Master Variation Ratio Curve (MVRC)."

The MVRC is a function represented by the following Equation 4:

$$[\text{Equation 4}]$$

$$\text{MVRC (concentration)} = \text{Estimated optical signal value/Measured optical signal value}$$

wherein the estimated optical signal value is an optical signal value estimated by MCC for a sample with a corresponding concentration; and the measured optical signal value is an optical signal value practically measured for the same sample.

The foregoing MCC is a function represented by the following Equation 5:

$$[\text{Equation 5}]$$

$$\text{Concentration} = \text{MCC (estimated optical signal value)}$$

As an inverse function of the MCC, iMCC is represented by the Equation 6:

$$[\text{Equation 6}]$$

$$\text{Estimated optical signal value} = \text{iMCC (concentration)}$$

Using Equation 4, 5 and 6, the following Equation 7 may be produced:

$$[\text{Equation 7}]$$

$$\text{Estimated optical signal value} = \text{Measured optical signal value} \times$$

$$\text{MVRC (concentration)} = \text{iMCC (concentration)}$$

In Equation 7, both sides of the second equal sign also become a concentration equation represented by the following Equation 8. Solving Equation 8, an unknown concentration of a sample may be calculated from a measured optical signal value of the same sample. For a case with difficulty in mathematical calculation, the unknown concentration may be obtained by altering a concentration of the sample to trace a value close to the true solution of the equation (that is, the unknown concentration), although mathematically solving Equation 8 results in a solution for the unknown concentration.

[Equation 8]

$$\text{Measured optical signal value} \times \text{MVRC (concentration)} = \text{iMCC (concentration)}$$

An unknown concentration of a sample may be accurately calculated by selecting an MVRC containing the same number of undetermined coefficients as the number of samples with known concentration, which are used in operations S10 and S20, and by calculating the undetermined coefficients. This procedure may be automatically executed through a calibration program installed in a test device.

Moreover, the following Equation 9 is derived from Equation 8. A measured optical signal value for a random concentration is calculated by both of the MCC and iMCC equations, respectively, or otherwise, a true optical signal value is obtained by practical measurement. As a result, a plurality of data comprising a concentration paired with a corresponding measured optical signal value (calculated or practically measured) is provided (S40).

[Equation 9]

$$\text{Measured optical signal value (or true optical signal value)} = \text{iMCC (concentration)/MVRC (concentration)}$$

From Equation 9, plural pairs of concentrations and corresponding measured or true optical signals for given concentrations are utilized to calibrate an MCC distributed by a manufacturer. That is, a least mean square (LMS) method is used with a pair of concentrations and corresponding optical signals calculated above, thereby calibrating a coefficient of the MCC. Next, after storing the calibrated MCC, the stored MCC is used to calculate a concentration of a sample from a measured optical signal value of the sample during experimentation.

The aforementioned methods may be carried out by a computer program product embodied on a computer readable medium, the computer program product, in one exemplary embodiment, being executed by a computer with a processor and a memory.

As a result of estimating a concentration according to an exemplary embodiment, it was found that the estimated value is very close to a measured true concentration, thus enhancing accuracy of test results. The calibration method according to the exemplary embodiments may be effectively employed in calibration of test results by a large-scale apparatus, without being limited to a compact analytic device for a test device.

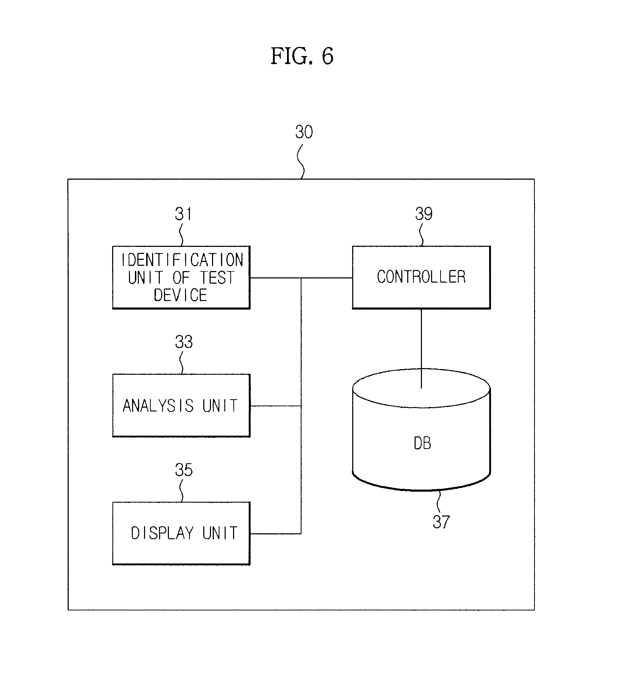

FIG. 6 illustrates a block diagram of an analytic device for using spectrophotometric analysis to analyze a sample placed in a test device, and for calibrating the analyzed results, according to one exemplary embodiment. The analytic device 30 includes an identification unit 31 which recognizes a test device mounted on the analytic device and to recognize an ID of the test device; a database 37 which stores information about test items executable in the test device depending on the ID of the test device, types of reagents contained in the test device, etc.; an analysis unit 33 which irradiates a reaction product and to analyze optical signals such as light absorbance or light transmittance, thereby completing qualitative or quantitative analysis of a sample; a display unit 35 which displays the analyzed results to a user; and a controller 39 which controls operation of the foregoing elements of the analytic device 30.

The analysis unit 33 measures an optical signal for a sample with a known concentration which was introduced into the test device. The analysis unit 33 then defines a relationship between a concentration of the sample and a ratio of an estimated optical signal value from the known sample concentration to the measured optical signal value. Such a relationship and, in addition, an MCC and a measured optical signal value for a sample with unknown concentration, are used to calculate the unknown concentration of the sample. The analysis unit 33 may also serve to calculate each of the measured optical signals corresponding to plural different concentrations using the foregoing MCC and the defined relationship. The analysis unit 33 may also calibrate a coefficient of the MCC, based on the calculated plural concentrations and corresponding optical signal values thereof. Consequently, using the MCC with the calibrated coefficient may more accurately calculate an unknown concentration of a sample from a measured light absorbance of the same sample.

Although a few exemplary embodiments have been shown and described, it would be appreciated by those skilled in the art that substitutions, variations and/or modifications may be made in these embodiments without departing from the principles and spirit of the inventive concept, the scope of which is defined in the claims and their equivalents.

**Claims**

1.  A method for calibrating results of a test device, the method comprising:

    measuring an optical signal for a sample contained in the test device to obtain a measured optical signal value, the sample having a known concentration; and
    determining a relationship between the known concentration of the sample and a ratio of an estimated optical signal value to the measured optical signal value of the sample.

2.  The method according to claim 1, wherein the measuring the optical signal comprises measuring optical signals for a plurality of samples contained in the test device to obtain a plurality of measured optical signal values, the plurality of samples having different concentrations.

3.  The method according to claim 1, wherein the estimated optical signal value is calculated on a master calibration curve (MCC) as a mathematical function expressing an interrelation between a sample concentration and a light absorbance at the sample concentration.

4.  The method according to claim 3, wherein the interrelation is represented by a relationship in a curve equation form.

5.  The method according to claim 4, wherein the curve equation is obtained by applying each of the sample concentration, the estimated optical signal value and the measured optical signal value in the relationship expressed in the form of the curve equation with a number of undetermined coefficients, and then, determining values of the number of undetermined coefficients of the curve equation.

6.  The method according to claim 5, wherein the number of the undetermined coefficients is substantially the same as a number of samples contained in the test device.

7.  The method according to claim 5, further comprising calculating an unknown concentration of a sample using an optical signal value measured for the sample with the unknown concentration, the determined relationship and the MCC.

8.  The method according to claim 5, wherein the calculating the unknown concentration of the sample is performed using the following equation, and the unknown concentration is a solution obtained by solving the equation or, otherwise, by altering the concentration to trace a concentration near the correct solution:

$$\text{Measured optical signal value} \times \text{MVRC (concentration)} = \text{iMCC (concentration)}$$

    wherein the measured optical signal value is an optical signal value measured for a sample with an unknown concentration;
    MVRC is a master variation ratio curve and is the determined relationship defined above; and
    iMCC is an inverse function of the MCC.

9.  The method according to claim 3, further comprising calculating each of the plurality of measured optical signal values using the MCC and the determined relationship or by using practical measurement of each of the plurality of measured optical signal values.

10. The method according to claim 9, wherein the calculating each of the plurality of measured optical signal values is performed using the following :

$$\text{Measured optical signal value} = iMCC \text{ (concentration)} /$$

$$MVRC \text{ (concentration)}$$

wherein MVRC is the master variation ratio curve and is the determined relationship defined above; and iMCC is an inverse function of the MCC.

**11.** The method according to claim 9, further comprising calibrating a coefficient of the MCC based on a plurality of pairs of concentrations and a plurality of optical signal values corresponding to the concentrations.

**12.** The method according to claim 11, further comprising calculating an unknown concentration of at least one of the samples by applying the MCC with the calibrated coefficient to the measured optical signal value of at least one of the samples.

**13.** A computer program product calibrating the results of a test device, the computer program product embodied on a computer readable medium and when executed by a computer, performing the method of:

measuring an optical signal for a sample contained in the test device to obtain a measured optical signal value, the sample having a known concentration; and
determining a relationship between the known concentration of the sample and a ratio of an estimated optical signal value to the measured optical signal value of the sample.

**14.** An device for analyzing a sample contained in a test device, comprising:

an identification unit which recognizes the test device; and
an analysis unit which analyzes the sample contained in test device using spectrophotometric analysis to measure an optical signal for the sample and determines a relationship between a concentration of the sample and a ratio of an estimated optical signal value to the measured optical signal value of the sample.

**15.** The device according to claim 14, wherein the analysis unit calculates each of a plurality of measured optical signal values corresponding to a plurality of different concentrations using a master calibration curve (MCC), as a mathematical function expressing an interrelation between a sample concentration and a light absorbance of the sample concentration, as well as the determined relationship, and calibrates a coefficient of the MCC based on the calculated plurality of concentrations and corresponding optical signal values thereof.

FIG. 1

FIG. 2

FIG. 3

FIG. 4

```
        ┌──────────────┐
        │    START     │
        └──────┬───────┘
               │
               ▼
┌──────────────────────────────────┐
│   INTRODUCTION OF SAMPLE WITH     │ ～S10
│       KNOWN CONCENTRATION         │
└──────────────┬───────────────────┘
               │
               ▼
┌──────────────────────────────────┐
│    MEASUREMENT OF OPTICAL SIGNAL  │ ～S20
└──────────────┬───────────────────┘
               │
               ▼
┌──────────────────────────────────┐
│  DEFINITION OF RELATIONSHIP BETWEEN │
│  CONCENTRATION AND RATIO OF ESTIMATED │ ～S30
│   OPTICAL SIGNAL VALUE TO MEASURED │
│        OPTICAL SIGNAL VALUE       │
└──────────────┬───────────────────┘
               │
               ▼
┌──────────────────────────────────┐
│  CALCULATION OF OPTICAL SIGNAL VALUE │
│     CORRESPONDING TO RANDOM       │ ～S40
│ CONCENTRATION BY INVERSE FUNCTION OF │
│   MCC AND DEFINED RELATIONSHIP    │
└──────────────┬───────────────────┘
               │
               ▼
┌──────────────────────────────────┐
│   ESTIMATION OF CALIBRATED MCC    │ ～S50
└──────────────┬───────────────────┘
               │
               ▼
        ┌──────────────┐
        │     END      │
        └──────────────┘
```

# FIG. 5A

ESTIMATED OPTICAL SIGNAL VALUE/
MEASURED OPTICAL SIGNAL VALUE

# FIG. 5B

ESTIMATED OPTICAL SIGNAL VALUE/
MEASURED OPTICAL SIGNAL VALUE

FIG. 6

30

31

| IDENTIFICATION UNIT OF TEST DEVICE |

39

| CONTROLLER |

33

| ANALYSIS UNIT |

35

| DISPLAY UNIT |

DB

37